Europäisches Patentamt

(19) European Patent Office

Office européen des brevets

(11) Publication number: **0 021 242**
**B1**

(12) **EUROPEAN PATENT SPECIFICATION**

(45) Date of publication of patent specification: 07.04.82

(21) Application number: 80103241.8

(22) Date of filing: 11.06.80

(51) Int. Cl.³: **C 07 C 69/80,**
**C 07 C 67/08,**
**C 07 C 69/44,**
**C 07 C 69/76, C 07 C 69/48**

(54) **Use of microwave energy in the production of plasticizer esters.**

(30) Priority: 20.06.79 US 50492

(43) Date of publication of application:
07.01.81 Bulletin 81/1

(45) Publication of the grant of the patent:
07.04.82 Bulletin 82/14

(84) Designated Contracting States:
AT BE DE FR GB NL

(56) References cited:
DE - A - 1 593 712
DE - A - 2 025 608

(73) Proprietor: BASF Canada Inc.
5850 Côte de Liesse
Town of Mount-Royal (CA)

(72) Inventor: Bhargava, Naresh
1106 Edythe Avenue
Cornwall Ontario (CA)

(74) Representative: Michaelis, Wolfgang, Dr.
c/o BASF Aktiengesellschaft 38 Carl-Bosch-
Strasse
D-6700 Ludwigshafen (DE)

Courier Press, Leamington Spa, England.

## Use of microwave energy in the production of plasticizer esters

The present invention is concerned with an improved process for the production of plasticizer esters. More particularly, the invention is directed to the use of microwave energy in esterification reactions to accelerate the rate of reaction and upgrade the quality of ester type of plasticizers.

Esters of aliphatic or aromatic di- or tricarboxylic acids and aliphatic alcohols are used extensively in the plastic industry as PVC plasticizer and are manufactured in large amounts. It is known that these esters can be prepared by reaction of the acids or their anhydrides with the alcohols at elevated temperature in the presence or absence of an esterification catalyst and/or azeotropic entrainer.

Various catalysts have been used to increase the speed of reaction, which include sulfuric acid, toluene sulfonic acid, phosphoric acid, organo tin compounds such as dibutyl tin maleate, organo titanium compounds such as tetra n-butyl titanate and aluminum compounds such as aluminium oxide.

It is common practice to use either alcohol or acid in excess and monitor the conversion of the other. In the case of esterification reactions without catalyst a conversion of about 98% can be achieved in about 10 hours. With the use of catalyst the reaction can be driven to about 99.9% in about 3.0 hours. However, most of the time it is stopped at a conversion of about 99.0% and the crude product is refined with a caustic solution followed by stripping of excess alcohol. During the reaction, there is always degradation of alcohol, leading to the formation of alkenes, ethers, aldehydes, etc.

In the manufacture of plasticizers where an excess of alcohol is utilized, the reaction must be followed by neutralization, stripping and excess alcohol, charcoal treatment and filtration. Stripping of alcohol is usually carried out at low pressures and with the help of steam. During the stripping operation, the free alcohol and other lower boiling impurities drop fast initially but require long stripping time to remove last traces. It is common to obtain products with 0.1 to 0.2% of alcohol/low boiling impurities after 7—8 hours of stripping. The product also must be dried to remove water and typically the moisture content runs around 300 ppm. Recently, the demand for high quality materials for use in medical, food and capacitor application is increasing where free alcohol and moisture must be brought down in the range of I10 ppm and 50 ppm, respectively. Also there are requirements for high electrical properties.

It is therefore an object of the invention to provide an esterification process, continuous or batch alike, which has very short reaction times, achieves high conversion of reactants, reduces the degradation of materials and results in plasticizer esters having significantly low contents of free alcohol and moisture.

This object is achieved according to this invention in an improved process of preparing plasticizer esters by reacting an aliphatic or aromatic dicarboxylic or tricarboxylic acid or an anhydride thereof and an aliphatic alcohol of 1 to 15 carbon atoms while continuously removing the water of reaction, wherein the improvement comprises carrying out the esterification under irradiation with microwaves.

In essence, the invention is directed to the use of microwave energy in a process involving reaction of one or more organic chemical compounds where one of the reaction products is polar and has a dielectric constant of over 4.0.

In an esterification reaction, one brings the alcohol molecule in contact with an acid/anhydride/half ester molecule in the presence or absence of a catalyst and at a particular energy level. This energy level is achieved by heating the molecules by conventional means. On reaction, a water molecule is formed. As the reaction is reversible, this molecule is removed by distillation of the water-alcohol azeotrope to prevent the reaction from going backwards. In a conventional heating system, the energy is transferred from the heating medium through a conductor and a temperature gradient in the material exists.

It has now been found quite surprisingly that microwaves are much more efficient and faster in transferring energy to the reactants and can be employed in the above reaction to accelerate the rate of reaction by about 4 times.

This came as a surprise since microwaves have so far been used only in communication, medicine, in food processing and in modifying of starch. In the case of esterification, the reaction can easily be carried out to achieve over 99.9% conversion of acid/anhydride with non acid catalysts which eliminates the requirements of alkali refining for the manufacture of commercial grade esters. As the reaction time is very short, the degradation of the alcohol is minimized. Reduction in degradation of alcohol and elimination of alkali refining reduces the product losses and improves the yield.

It has also been found that when a commercial grade plasticizer or similar material is exposed to microwaves under reduced pressure, the free alcohol and moisture contents are substantially reduced, generally to below 10 ppm and 50 ppm, respectively.

The microwaves suitable for application in the process according to the invention are electromagnetic waves having frequencies ranging from 30 to 300,000 MHz. For industrial applications, however, only certain specific frequency bands are permitted by the authorities in several countries. These frequency bands include 40—50, 896, 915 and 2450 MHz. In practice, these frequencies will be applicable in the process of the invention. It is to be understood, however, that the invention is not limited to these specific frequencies.

Esterification of the starting material is carried out in the conventional manner in the presence or absence of an esterification catalyst and preferably in the absence of entrainers while returning the excess of alcohol used, the excess being about up to 2 miles and preferably up to 1 mole of alcohol per mole of acid or anhydride.

Examples of suitable aliphatic and aromatic di- or tri-carboxylic acids or their anhydrides are adipic acid, phthalic anhydride, maleic anhydride, fumaric acid, malonic acid, succinic acid, glutaric acid, azelaic acid, sebacic acid, and particularly phthalic anhydride, trimellitic anhydride and adipic acid. Examples of alcohols of one to 15 carbon atoms are methanol, ethanol, butanol, n-octanol-(1), n-octanol-(2), 2-ethylhexanol-(1), n-nonyl alcohol, isononyl, alcohol, decanol, isodecanol and iso tridecanol.

Generally, the reaction temperatures range from 60° to 300°C, the preferred temperatures being between 150° and 250°C.

Preferred embodiments of the invention will now be described in greater detail with reference to the following examples and accompanying drawings, wherein:

Figure 1 schematically represents a microwave oven which has been modified to accommodate an esterification apparatus; and

Figure 2 is a diagram illustrating the conversion rate in time for the production of dioctyl phthalate (DOP).

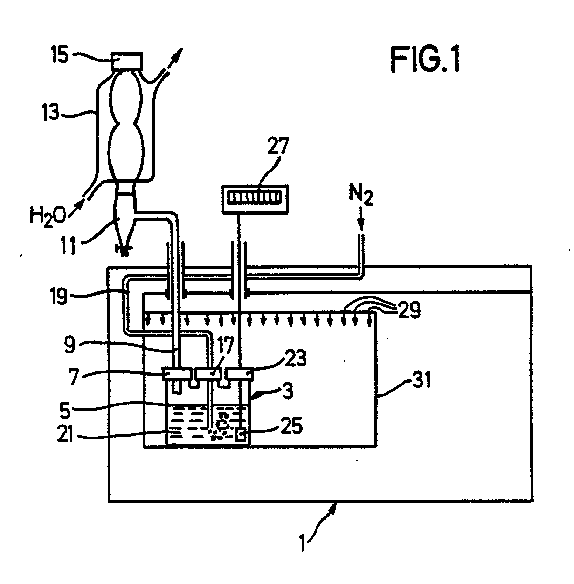

As shown in Figure 1, a microwave oven 1 operating at 2450 MHz and with a power source of 700 watts is equipped with an esterification apparatus 3. The apparatus consists of a 3 neck jar 5 of one liter capacity. Neck 7 is connected to a column 90 which leads the vapours from the jar out of the microwave oven to a Stark and Dean trap 11 mounted with a water cooled condenser 13, the outlet 15 of the condenser being connected to a vacuum source. The second neck 17 is connected to a nitrogen source through conduit 19 so as to permit nitrogen gas to bubble through the reaction mixture 21 in the jar. The third neck 23 is used to accommodate a temperature sensing device 25 connected to a temperature recorder 27. Precautions are of course taken to prevent any leakage of microwaves 29. The oven door 31 provided with a viewing screen enables one to supervise the reaction as it takes place inside the microwave oven.

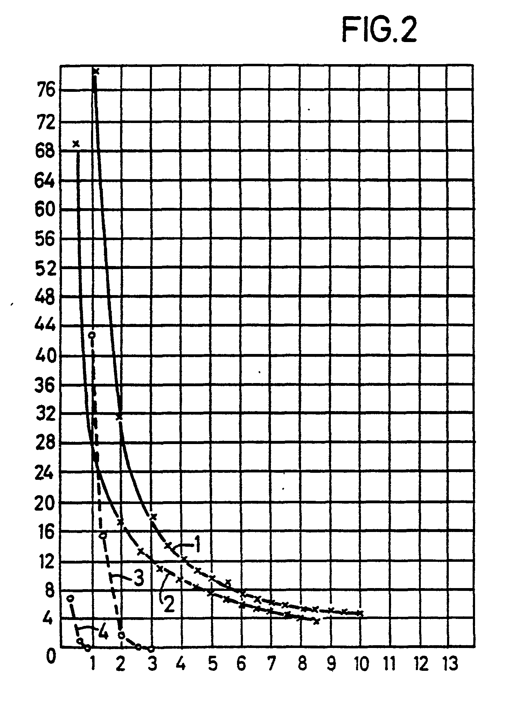

In Figure 2, there is represented a plot of the acid number against the reaction time for the production of DOP carried out in the absence of catalyst and microwaves (curve 1), in the absence of catalyst but in the presence of microwaves (curve 2), in the presence of catalyst but no microwaves (curve 3) and, finally, in the presence of both catalyst and microwaves (curve 4). As it is apparent from this diagram, exposure to microwaves greatly increases the rate of conversion (or, reduces the reaction time) with the effect being more pronounced in the presence of catalyst (curve 4).

The following examples illustrate the invention:

### Example 1 (Comparison)

148g of phthlatic anhydride and 200 g of 2-ethylhexanol were charged in the apparatus 3 shown in Figure 1 but outside the microwave oven and the jar was heated electrically. A further addition of 60 g and 30 g of 2-ethylhexanol was made after 10 and 20 minutes. Rection proceeded with the splitting of water molecules which were removed in the trap. Reaction temperature varied between 200—240°C. Progress of the reaction was monitored by measuring the acid number. At the end of 10 hours, an acid number of 5.6 was achieved. This is equivalent to 97.9% conversion of phthalic anhydride.

### Example 2

148 g of phthalic anhydride and 200 g of 2-ethylhexanol were charged in the apparatus 3 shown in Figure 1 and reacted in the presence of microwaves. A further addition of 60 g and 30 g 2-ethylhexanol was made after 10 and 20 minutes. Temperature was maintained between 200—240°C. After 8.5 hours, an acid number of 4.5 was achieved which is equivalent to about 98.2% conversion of phthalic anhydride.

### Example 3 (Comparison)

148 g of phthalic anhydride, 200 g of 2-ethylhexanol and 0.2 g of tetrabutyl titanate were charged in the apparatus 3 shown in Figure 1 but outside the microwave oven and heated electrically. A further addition of 60 g and 30 g 2-ethylhexanol was made after 15 and 21 minutes. Temperature was maintained between 200 and 240°C. After 3 hours, an acid number of 0.08 was achieved which is equivalent to a conversion of 99.97%.

### Example 4

148 g of phthalic anhydride, 200 g of 2-ethylhexanol and 0.2 g tetrabutyl titanate were charged in the apparatus 3 shown in Figure 1 and exposed to microwaves. Reaction started with the distillation of water which was condensed and collected in the trap. After 15 and 23 minutes exposure, a further addition of 60 g and and 30 g of 2-ethylhexanol was made to the reactants. After 80 minutes exposure, an acid number of 0.09 was obtained which is equivalent to 99.97% conversion of phthalic anhydride.

### Example 5 (Comparison)

148 g of phthalic anhydride and 200 g of 2-ethylhexanol were charged along with 1.2 g of tetrabutyl titanate in the apparatus 3 shown in Figure 1 and heated electrically. A further addition of 60 g and 30 g 2-ethylhexanol was made after 15 and 21 minutes. Temperature was maintained to a maximum of 240°C. After 3 hours reaction, a final acid number of 0.09 was achieved which is equivalent to 99.97% conversion of phthalic anhydride.

### Example 6

148 g of phthalic anhydride, 200 g of 2-ethylhexanol and 1.2 g of tetrabutyl titanate were charged in the apparatus 3 shown in Figure 1 and exposed to microwaves. Reaction started with the distillation of water which was condensed and collected in the trap. After 15 and 21 minutes exposure, a further addition of 60 g and 30 g of 2-ethylhexanol was added to the reactants. Reaction temperature was kept between 200 and 265°C. Reaction was complete in 45 minutes when an acid number of 0.09 was achieved. This is equivalent to a conversion of 99.97% of phthalic anhydride.

### Example 7

148 g of phthalic anhydride and 74 g butanol were charged along with 1.2 g of toluene sulfonic acid in the apparatus 3 shown in Figure 1 and exposed to microwaves. After 2.5, 12.5 and 22.5 minutes exposure, an addition of 70 g, 60 g and 30 g 2-ethylhexanol was made. Reaction temperature was controlled between 160 and 210°C. At the end of the 56 minutes exposure, an acid number of 1.12 was achieved. this is equivalent to over 99.9% conversion of phthalic anhydride.

### Example 8

146 g adipic acid, 195 g of 2-ethylhexanol and 0.2 g tetrabutyl titanate were charged in the apparatus 3 shown in Figure 1 and exposed to microwaves. After 6 and 11 minutes exposure, an additional 57 ml and 50 ml of 2-ethylhexanol was charged and exposure continued for a total of 66 minutes. Final acid number was 0.06. This is equivalent to 99.98% of adipic acid.

### Example 9

148 g of phthalic anhydride, 379 g isodecanol and 0.2 g tetrabutyl titanate were charged in the apparatus 3 shown in Fig. 1 and exposed to microwaves. After 55 minutes of exposure, the acid number of the crude product was 0.08. This is equivalent to 99.96% conversion of phthalic anhydride.

### Example 10

130 g of trimellitic anhydride, 316 g of 2-ethylhexanol and 0.2 g of tetrabutyl titanate were charged in the apparatus 3 shown in Fig. 1 and exposed to microwaves. After 71 minutes exposure, the final acid number was 0.08. This is equivalent to 99.97% conversion of trimellitic anhydride.

### Example 11

166 g azelaic acid, 200 g 2-ethylhexanol and 0.5 g of tetrabutyl titanate were charged in the apparatus 3 shown in Fig. 1 and exposed to microwaves. Reaction temperature was controlled between 155 and 245°C. Additional 59 g 2-ethylhexanol was added after 15 minutes exposure. After 65 minutes exposure, an acid number of 0.09 was achieved.

### Example 12

A commercial microwave oven was modified so that nitrogen supply and vacuum were available inside the oven. 500 grams of DOP were exposed to microwaves (2450 MHz and 700 watts) at about 30 mm. pressure for 3 minutes. The impurities started boiling and were extracted by vacuum. The sample was allowed to cool for 2 minutes and then reexposed for 3 minutes. The product was cooled and analysed as follows:

|  | Original | Exposed |
|---|---|---|
| Moisture ppm | 350 | 20 |
| Alcohol content | 0.1% | 0.04% |
| Cell V.R.[1] at 25°C | $14.7 \times 10^{11}$ ohm-cm | $28.2 \times 10^{11}$ ohm-cm |

Example 13

500 grams of DOP were exposed to microwaves at high power setting for 5 minutes followed by 5 minutes at medium low setting with nitrogen purge and at about 30 mm pressure. The product was cooled and analysed as follows:

|  | Original | Exposed |
| --- | --- | --- |
| Moisture ppm | 310 | 10 |
| Alcohol | 0.05% | not detectable |
| Cel V.R. as is | $12.4 \times 10^{11}$ ohm-cm | $12.4 \times 10^{11}$ ohm-cm |
| Cell V.R. after filtration | $15.8 \times 10^{11}$ ohm-cm | $22.6 \times 10^{11}$ ohm-cm |

Example 14

Example 13 was repeated, with the following results:

|  | Original | Exposed |
| --- | --- | --- |
| Moisture ppm | 310 | 40 |
| Alcohol | 0.05% | not detectable |
| Cell V.R. as is | $12.4 \times 10^{11}$ ohm-cm | $11.3 \times 10^{11}$ ohm-cm |
| Cell V.R. after filtration | $15.8 \times 10^{11}$ ohm-cm | $19.2 \times 10^{11}$ ohm-cm |

(1) Cell Volume Resistivity

**Claims**

1. A process of preparing plasticizer esters by reacting an aliphatic or aromatic dicarboxylic or tricarboxylic acid or an anhydride thereof and an aliphatic alcohol of 1 to 15 carbon atoms while continuously removing the water of reaction, characterised in that the esterification is carried out under irradiation with microwaves.

2. A process as claimed in claim 1, characterised in that the frequency of microwaves is between 30 and 300,000 MHz.

3. A process as claimed in claim 2, characterised in that the frequency of microwaves is 40—50 MHz.

4. A process as claimed in claim 2, characterised in that the frequency of microwaves is 896 MHz.

5. A process as claimed in claim 3, characterised in that the frequency of microwaves is 915 or 2450 MHz.

6. A process as claimed in claim 1, characterised in that the reaction is carried out in the presence of an esterification catalyst.

7. A process as claimed in claim 1, characterised in that the reaction is carried out a temperature of 60° to 300°C.

8. A process as claimed in claim 7, characterised in that the reaction temperature is between 150°C and 250°C.

9. A process as claimed in claim 1, characterised in that the alcohol is selected from the group consisting of methanol, ethanol, butanol, n-octanol-(1), n-octanol-(2), 2-ethylhexanol-(1), n-nonyl alcohol, isononyl alcohol, decanol and isodecanol.

10. A process as claimed in claim 9, characterised in that the alcohol is butanol, 2-ethylhexanol-(1) or isodecanol.

11. A process as claimed in claim 1, characterised in that the dicarboxylic acid is adipic acid.

12. A process as claimed in claim 1, characterised in that the dicarboxylic acid anhydride is phthalic anhydride.

13. A process as claimed in claim 1, characterised in that the tricarboxylic acid is trimellitic acid.

14. A process as claimed in claim 1, characterised in that the alcohol is used in excess over the stoichiometric quantity required for the reaction.

15. A process as claimed in claim 14, characterised in that the alcohol is used in an excess of up to 2 miles per mole of acid or anhydride.

16. A process of upgrading the quality of ester plasticizers, characterised by exposition of the plasticizer to microwaves under reduced pressure, whereby the free alcohol and moisture contents are substantially reduced.

**0 021 242**

Revendications

1. Procédé pour la préparation d'esters plastifiants par la mise en réaction d'un acide bicarboxylique ou tricarboxylique aliphatique ou aromatique ou un anhydride de celui-ci et d'un alcool aliphatique comportant 1 à 15 atomes de carbone, tout en éliminant en permanence l'eau réaction, caractérisé en ce que l'estérification est effectuée sous irradiation par des micro-ondes.

2. Procédé selon la revendication 1, caractérisé en ce que la fréquence des micro-ondes est comprise entre 30 et 300 000 MHz.

3. Procédé selon la revendication 2, caractérisé en ce que la fréquence des micro-ondes est de 40 à 50 MHz.

4. Procédé selon la revendication 2, caractérisé en ce que la fréquence des micro-ondes est de 896 MHz.

5. Procédé selon la revendication 3, caractérisé en ce que la fréquence des micro-ondes est de 915 ou de 2450 MHz.

6. Procédé selon la revendication 1, caractérisé en ce que la réaction est menée en présence d'un catalyseur d'estérification.

7. Procédé selon la revendication 1, caractérisé en ce que la réaction est menée à une température comprise entre 60° et 300°C.

8. Procédé selon la revendication 7, caractérisé en ce que la température de réaction est comprise entre 150°C et 250°C.

9. Procédé selon la revendication 1, caractérisé en ce que l'alcool est choisi dans le groupe constitué par le méthanol, l'éthanol, le butanol, le n-octanol-(1), le n-octanol-(2), le 2-éthylhexanol-(1), l'alcool n-nonylique, l'alcool isononylique, le décanol et l'isodécanol.

10. Procédé selon la revendication 9, caractérisé en ce que l'alcool est le butanol, le 2-éthylhexanol-(1) ou l'isodécanol.

11. Procédé selon la revendication 1, caractérisé en ce que l'acide bicarboxylique est l'acide adipique.

12. Procédé selon la revendication 1, caractérisé en ce que l'anhydride d'acide bicarboxylique est l'anhydride phtalique.

13. Procédé selon la revendication 1, caractérisé en ce que l'acide tricarboxylique est l'acide trimellitique.

14. Procédé selon la revendication 1, caractérisé en ce que l'alcool est utilisé en excès par rapport à la quantité stoechiométrique nécessaire pour la réaction.

15. Procédé selon la revendication 14, caractérisé en ce que l'alcool est utilisé en un excès qui peut atteindre 2 mol par mol d'acide ou d'anhydride.

16. Procédé pour améliorer la qualité de plastifiants du type des esters, caractérisé par l'exposition du plastifiant à des micro-ondes sous pression réduite, ce qui réduit considérablement les teneurs en alcool libre et en humidité.

**Patentansprüche**

1. Ein Verfahren zur Herstellung von Weichmacherestern durch Reaktion einer aliphatischen oder aromatischen Dicarbon- oder Tricarbonsäure oder eines entsprechenden Anhydrides mit einem aliphatischen Alkohol mit 1 bis 15 Kohlenstoffatomen unter kontinuierlicher Entfernung des Reaktionswassers, dadurch gekennzeichnet, daß die Veresterung unter Mikrowellenbestrahlung durchgeführt wird.

2. Ein Verfahren gemäß Anspruch 1, dadurch gekennzeichnet, daß die Mikrowellenfrequenz zwischen 30 und 300 000 MHz liegt.

3. Ein Verfahren gemäß Anspruch 2, dadurch gekennzeichnet, daß die Mikrowellenfrequenz 40—50 MHz beträgt.

4. Ein Verfahren gemäß Anspruch 2, dadurch gekennzeichnet, daß die Mikrowellenfrequenz 896 MHz beträgt.

5. Ein Verfahren gemäß Anspruch 3, dadurch gekennzeichnet, daß die Mikrowellenfrequenz 915 oder 2450 MHz beträgt.

6. Ein Verfahren gemäß Anspruch 1, dadurch gekennzeichnet, daß die Reaktion in Anwesenheit eines Veresterungskatalysators durchgeführt wird.

7. Ein Verfahren gemäß Anspruch 1, dadurch gekennzeichnet, daß die Reaktion bei einer Temperatur zwischen 60 und 300°C durchgeführt wird.

8. Ein Verfahren gemäß Anspruch 7, dadurch gekennzeichnet, daß die Reaktionstemperatur zwischen 150°C und 250°C liegt.

9. Ein Verfahren gemäß Anspruch 1, dadurch gekennzeichnet, daß der Alkohol aus der aus Methanol, Äthanol, Butanol, n-Octanol-(1), n-Octanol-(2), 2-Äthylhexanol-(1), n-Nonylalkohol, Isononylalkohol, Decanol und Isodecanol bestehenden Gruppe ausgewählt wird.

10. Ein Verfahren gemäß Anspruch 9, dadurch gekennzeichnet, daß der Alkohol Butanol, 2-Äthylhexanol-(1) oder Isodecanol ist.

11. Ein Verfahren gemäß Anspruch 1, dadurch gekennzeichnet, daß die Dicarbonsäure Adipinsäure ist.

12. Ein Verfahren gemäß Anspruch 1, dadurch gekennzeichnet, daß das Dicarbonsäureanhydrid Phthalsäureanhydrid ist.

13. Ein Verfahren gemäß Anspruch 1, dadurch gekennzeichnet, daß die Tricarbonsäure Trimellitsäure ist.

14. Ein Verfahren gemäß Anspruch 1, dadurch gekennzeichnet, daß Alkohol im Überschuß über der für die Reaktion benötigten stöchiometrischen Menge eingesetzt wird.

15. Ein Verfahren gemäß Anspruch 14, dadurch gekennzeichnet, daß der Alkohol in einem Überschuß von bis zu 2 Mol pro Mol Säure oder Anhydrid eingesetzt wird.

16. Ein Verfahren zur Qualitätsverbesserung von Weichmacherestern, gekennzeichnet durch eine Bestrahlung des Wiechmachers mit Mikrowellen unter vermindertem Druck, wodurch die Gehalte an freiem Alkohol und Feuchtigkeit wesentlich vermindert werden.

FIG.1

FIG.2